# EUROPÄISCHE PATENTSCHRIFT

(11) **EP 1 008 593 B1**
(45) Veröffentlichungstag und Bekanntmachung des Hinweises auf die Patenterteilung: **10.05.2006**
(21) Anmeldenummer: 99123929.4
(22) Anmeldetag: 02.12.1999
(51) Int. Cl.: C07D 307/58, A61K 8/18, A61Q 5/02, A61Q 17/04

(54) **Benzyliden-gamma-butyrolactone, Verfahren zu ihrer Herstellung und ihre Verwendung als UV-Absorber**
Benzylidene-gamma-butyrolactones, process for their production and use thereof as UV absorbers
Benzylidène-gamma-butyrolactone, leur procédé de préparation et leur utilisation comme absorbeurs d'UV

(30) Priorität: 11.12.1998 DE 19857252
(43) Veröffentlichungstag der Anmeldung: 14.06.2000
(73) Patentinhaber: Symrise GmbH & Co. KG, 37603 Holzminden (DE)
(72) Erfinder: Koch, Oskar, Dr., 37079 Göttingen (DE); Johncock, William, Dr., 37671 Höxter (DE); Langner, Roland, 37639 Bevern (DE)
(74) Vertreter: Eisenführ, Speiser & Partner

(56) Entgegenhaltungen:
- EP-A- 0 044 970
- EP-A- 0 368 717
- DE-A- 3 931 269
- P. RIOULT, J. VIALLE: "Composés organiques sulfurés XIV. Condensation des lactones, thiolactones et thiolannethiones-2 avec les aldéhydes aromatiques et le sulfure de carbone" BULL. SOC. CHIM. FR., Nr. 11, 1968, Seiten 4477-4483, XP000882402
- H. TORABI ET AL.: "The Reformatsky Reaction with Halolactones. I. Reaction with Nonsteroid Carbonyl Compounds" J. ORG. CHEM., Bd. 34, Nr. 12, 1969, Seiten 3792-3796, XP000882309
- K. MATUO, Y. HASUIKE: "Synthesis of 10-Aryl-3a,9-dihydro-1H,3H-furo[4,3-b][1,5 ]benzothiazepin-1-ones" CHEM. PHARM. BULL., Bd. 37, Nr. 10, 1989, Seiten 2803-2806, XP000882870
- H. ZIMMER ET AL.: "Substituted gama-Lactones. IV. Some Aldehyde Condensations with delta(beta, gamma)-Angelica- and gamma-Valerolactone" J. ORG. CHEM., Bd. 25, 1960, Seiten 838-839, XP000882308
- A. DATTA ET AL.: "Polarized ketene dithioacetals 63. Stereoselective synthesis of alpha-ylidene-gamma-butyrolactones" TETRAHEDRON, Bd. 43, Nr. 22, 1987, Seiten 5367-5374, XP000882871
- H. ZIMMER ET AL.: "Substituted gamma-Lactones. XIII. Nitration of Substituted alpha-Benzylidene-gamma-butyrolactones" J. ORG. CHEM., Bd. 29, 1964, Seiten 925-929, XP000882311

## Beschreibung

Die Erfindung betrifft modifizierte Benzyliden-γ-butyrolactone, ein Verfahren zu ihrer Herstellung und die Verwendung als UV-Absorber, beispielsweise in pharmazeutischen und kosmetischen Mitteln, Sonnenschutzmitteln, Tages- und Haarpflegeprodukten, zur Verbesserung von technischen Produkten, wie Anstriche, Lacke, Kunststoffe, Textilien, Verpackungsmaterialien und Kautschuken.

UV-Strahlen werden nach ihrer Wellenlänge unterteilt in UV-A-Strahlen (320-400 nm) und UV-B-Strahlen (280-320 nm). Die schädigende Wirkung, insbesondere das Auftreten von Sonnenbrand (Erythem), steigt neben der Dauer der Exposition mit sinkender Wellenlänge und ist damit bei der UV-B-Strahlung deutlich stärker ausgeprägt als bei der UV-A-Strahlung. Da Erytheme bereits nach kurzer, u. U. nach 20-30minütiger Sonnenexposition auftreten können, ist ein effizienter Schutz vor dieser Strahlung von besonderer Bedeutung.

Für diesen Bereich des UV-Schutzes sind bereits eine Reihe von UV-B-Absorbern aus dem Stand der Technik bekannt. Beispielhaft sind hier Campher-Derivate, Salicylsäure-Derivate, Benzophenone, Cinnamate, Benzimidazole sowie Triazine genannt. Ein Beispiel für Benzyliden-γ-butyrolactone ist in der europäischen Patentanmeldung EP-A 44970 beschrieben. Hierbei handelt es sich um Verbindungen der Formel 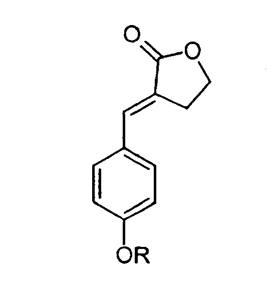 worin
- R: einen C₁-C₁₅-Alkylrest, verzweigt oder unverzweigt, Phenyl oder Benzyl darstellt.

Diese Verbindungen haben jedoch alle die Nachteile, daß sie entweder als Feststoff vorliegen und damit nur eine begrenzte Löslichkeit in den kosmetischen Zubereitungen besitzen, nur eine geringe Absorption zeigen oder eine Photostabilität aufweisen, die die erforderlichen Kriterien nicht erfüllt und somit nicht zufriedenstellend sind.

Die vorliegende Erfindung hat sich demgemäß die Aufgabe gestellt, verbesserte UV-B-Absorber zur Verfügung zu stellen.

Diese Aufgabe wird durch Benzyliden-γ-butyrolactone der allgemeinen Formel 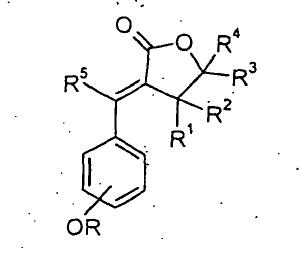 gelöst,
wobei
- R: Wasserstoff, oder C₁-C₆-Alkyl oder Cycloalkyl darstellt und
- R² und R⁵: unabhängig voneinander Wasserstoff oder C₁-C₈-Alkyl der Cycloalkyl darstellen und der Lactonring an R¹, R³ und R⁴ durch ein C₁-C₈-Alkyl trisubstituiert ist, wobei eine Alkylgruppe mindestens eine C₂-Kette beinhaltet. Bevorzugt sind Benzyliden-γ-butyrolactone, deren Benzylidenring in para-Stellung mit einem Methoxy-Rest substituiert ist und R¹ einen Ethylrest, R² ein Wasserstoff und R³/R⁴ einen Methylrest darstellen.

Eine bevorzugte Verbindung ist: p-Methoxybenzyliden-3,4,4-trimethyl-γ-butyrolacton.

Besonders bevorzugte Verbindungen sind:
p-Methoxybenzyliden-3-ethyl-4,4-dimethyl-γ-butyrolacton, p-Methoxybenzyliden-3,4-dimethyl-4-butyl-γ-butyrolacton, p-Methoxybenzyliden-3,4-dimethyl-4-ethyl-γ-butyrolacton.

Die erfindungsgemäßen Benzyliden-γ-butyrolactone lassen sich gemäß dem Stand der Technik durch Aldolkondensation von substituierten Benzaldehyden, beispielsweise Anisaldehyd, mit entsprechend substituierten γ-Butyrolactonen, beispielsweise β-Ethyl-γ,γ-dimethyl-γ-butyrolacton herstellen.

Die erfindungsgemäßen Benzyliden-γ-butyrolactone sind als UV-Absorber und Lösungsmittel für schlechter lösliche feste UV-Absorber innerhalb einer pharmazeutischen oder kosmetischen Zubereitung besonders geeignet. Gegenstand der Erfindung sind demgemäß auch Mittel, die die zuvor genannten Benzyliden-γ-butyrolactone enthalten und sich zum Schutz vor schädigender UV-Strahlung eignen. Dabei können die erfindungsgemäßen Mittel ein oder mehrere der erfindungsgemäßen Benzyliden-γ-butyrolactone enthalten.

Bevorzugt sind dabei Mittel, die 0,1 bis 15 Gew.-% an erfindungsgemäßen Benzy-Benzyliden-γ-butyrolactonen, bezogen auf das Gesamtgewicht der Zubereitung, enthalten. Besonders bevorzugt sind Mittel mit 1 bis 10 Gew.-%, höchst bevorzugt mit 2 bis 7 Gew.-% an erfindungsgemäßem Benzyliden-γ-butyrolacton.

Erfindungsgemäß eignen sich die zuvor genannten Mittel bevorzugt als Sonnenschutz- oder Tagespflegemittel zum Schutz der menschlichen Haut und Haare, insbesondere durch Dauerwelle, Färbung und Bleichen bereits vorgeschädigter Haare, vor schädigender UV-Strahlung.

Erfindungsgemäß können die Mittel auch mit an sich bekannten Zusatzstoffen und/oder bekannten UV-Absorbern anderer Substanzklassen und/oder bekannten Pigmenten vermischt werden.

Beispiele für gängige Zusatzstoffe sind Emulgatoren, grenzflächenaktive Verbindungen, Lanolin, Vaseline, Wasser, Triglyceride von Fettsäuren, Polyethylenglykole, Fettalkohole, ethoxylierte Fettalkohole, Fettsäureester wie z. B. Isopropylpalmitat, Isooctylstearat, Adipinsäurediisopropylester etc., natürliche oder synthetische Öle oder Wachse, Verdickungsmittel, wie z. B. Hydroxyethylcellulose, Bentonit etc., Konservierungsstoffe, Feuchtigkeitsmittel, Vitamine, skin-lightening-Wirkstoffe wie z.B. Hydrochinone, Arbutin, Kojisäure u. Derivate, Ascorbinsäure u. Derivate, Glutathion, Hydroxybenzaldoxime, z.B. 4-Hydroxy-3-methoxybenzaldehydoxim, Antioxidantien wie z.B. BHT, Vitamin-Derivate, Catechin-Derivate, z.B. Epigallocatechin-gallat oder 3,4-Dihydroxybenzaldehydoxim, Komplexbildner wie z.B. EDTA und Derivate, Insect-repellents wie z.B. DEET oder IR 3535, Siliconöle, Glycerin, Ethylalkohol und Parfumöle. Die Liste der Zusatzstoffe ist dabei nicht auf genannte Beispiele beschränkt.

Als Pigmente können z. B. Titandioxid, Zinkoxid, Perlglanzpigmente oder Farbpigmente beigemischt werden.

Beispiele für herkömmliche UV-Absorber umfassen p-Aminobenzoesäure, p-Aminobenzoesäureethylester (25 Mol) ethoxyliert, p-Dimethylaminobenzoesäure-2-ethylhexylester, p-Aminobenzoesäureethylester (2 Mol) N-propoxyliert, p-Aminobenzoesäureglycerinester, Salicylsäure-homomenthylester, Salicylsäure-2-ethylhexylester, Triethanolaminsalicylat, 4-Isopropylbenzylsalicylat, Anthranilsäuremethylester, Diisopropylzimtsäureethylester, p-Methoxyzimtsäure-2-ethylhexylester, Diisopropylzimtsäuremethylester, p-Methoxyzimtsäureisoamylester, p-Methoxyzimtsäure-diethanolaminsalz, p-Methoxyzimtsäure-isopropylester, 2-Ethylhexyl-2-cyano-3,3-diphenylacrylat, Ethyl-2-cyano-3,3'-diphenylacrylat, 2-Phenylbenzimidazolsulfonsäure und Salze, 3-(4'-Trimethylaminonium)-benzyliden-bornan-2-on-methylsulfat, Terephthalyliden-dibornansulfonsäure und Salze, 4-t-Butyl-4'-methoxy-dibenzoylmethan, β-Imidazol-4(5)-acrylsäure (Urocaninsäure), 2-Hydroxy-4-methoxybenzophenon, 2-Hydroxy-4-methoxybenzophenon-5-sulfonsäure, Dihydroxy-4-methoxybenzophenon, 2,4-Dihydroxybenzophenon, Tetrahydroxybenzophenon, 2,2'-Dihydroxy-4,4'-dimethoxybenzophenon, 2-Hydroxy-4-n-octoxybenzophenon, 2-Hydroxy-4-methoxy-4'-methylbenzophenon, 3-(4'-Sulfo)benzyliden-bornan-2-on und Salze, 3-(4'-Methylbenzyliden)-d,1-campher, 3-Benzyliden-d,1-campher, 4-Isopropyldibenzoylmethan, 2,4,6-Trianilino-(p-carbo-2'-ethylhexyl-1'-oxy)-1,3,5-triazin, Phenylen-bis-benzimidazyl-tetrasulfonsäure-dinatriumsalz und N-[(2 und 4)-[2-(oxobom-3-yliden)methyl]benzyl]-acrylamid-Polymer, Benzylidenmalonat-Polymer, 2,2'-Methylen-bis-(6-(2H-benzotriazol-2-yl)-4-(1,1,3,3-tetramethylbutyl)-phenol), 2,2'-(1,4-Phenylen)-bis-1H-benzimidazol-4,6-disulfonsäure, mononatriumsalz, 2,4-bis-((4-(2-Ethyl-hexyloxy)-2-hydroxy)-phenyl)-6-(4-methoxyphenyl)-(1,3,5)-triazin.

UV-Absorber, die sich zur Kombination mit den erfindungsgemäßen Mitteln als besonders geeignet herausgestellt haben, sind
p-Methoxyzimtsäure-2-ethylhexylester, p-Methoxyzimtsäure-isoamylester, 2-Phenylbenzimidazolsulfonsäure, 3-(4'-Methylbenzyliden)-d,1-campher, 2-Ethylhexyl-2-cyano-3,3-diphenylacrylat, Salicylsäure-2-ethylhexylester, 4-tert.-Butyl-4'-methoxydibenzoylmethan und Phenylen-bis-benzimidazyl-tetrasulfonsäure-dinatriumsalz, Indanyliden-Derivate gemäß DE 19631863 und Benzazol-Derivate gemäß DE 19648010.

Gegenstand der Erfindung ist ferner die Verwendung der erfindungsgemäßen Mittel in Kombination mit herkömmlichen UV-Absorbern zur Verstärkung des Schutzes vor schädigender UV-Strahlung über das Maß des Schutzes, der bei Einsatz gleicher Mengen herkömmlicher UV-Absorber allein oder der Mittel allein erzielt wird (synergistischer Effekt).

Weiterhin lassen sich die erfindungsgemäßen Mittel auch mit herkömmlichen UV-Absorbern kombinieren, die für den Schutz technischer Produkte, wie Anstriche, Lacke, Kunststoffe, Textilien, Verpackungsmaterialien oder Kautschuk eingesetzt werden.

Beispiele solcher UV-Absorber sind Verbindungen aus der Reihe der Benztriazole, Benzophenone, Triazine, Zimtsäureester sowie Oxalanilide.

Die erfindungsgemäßen Mittel können in alle heute üblichen kosmetischen oder pharmazeutischen Zubereitung vorliegen. Beispielsweise können sie als Emulsion, Milch, Lotion, Creme, Gel, Aerosol, Shampoo, Spülung, Kur oder Spray angeboten werden.

Überraschenderweise zeichnen sich die erfindungsgemäß Benzyliden-γ-butyrolacton enthaltenden Mittel durch eine Kombination an wünschenswerten Eigenschaften aus.

Die Mittel besitzen erfindungsgemäß die vorteilhaften Eigenschaften, daß sie eine hohe UV-Schutzleistung bei bereits niedrigen Einsatzkonzentrationen aufweisen, eine ausgezeichnete Licht- und Thermostabilität und eine gute Löslichkeit in kosmetischen Lösungsmitteln besitzen. Ferner besitzen die kristallinen, Öl-löslichen UV-Absorber erfindungsgemäß eine ausgezeichnete Löslichkeit in kosmetischen Lösungsmitten. Die Mittel weisen erfindungsgemäß eine gute Verträglichkeit mit kosmetischen Grundstoffen und eine gute pH-Stabilität auf, sind farblos, geruchsneutral und wasserfest. Ferner lassen sich die erfindungsgemäßen Mittel problemlos in kosmetischen Zubereitungen verarbeiten und zeigen eine gute Stabilität unter Anwendungsbedingungen sowie eine gute Verträglichkeit mit Verpackungsmaterialien. Darüber hinaus verursachen die erfindungsgemäßen Mittel keine Verfärbungen von Textilien und sind problemlos auswaschbar.

Im folgenden wird die Anmeldung unter Bezugnahme auf die Beispiele näher erläutert:

### 1. Erfindungsgemäßen Benzyliden-γ-butyrolactone und deren Herstellung:

Beispielhaft für die Herstellung der erfindungsgemäßen Benzyliden-γ-butyrolactone sei die Herstellung von p-Methoxybenzyliden-4-methyl-γ-butyrolacton (die Verbindung selbst ist nicht erfindungsgemäß) als Beispiel 1 näher erläutert.

### Beispiel 1: p-Methoxybenzyliden-4-methyl-γ-butyrolacton (nicht erfindungsgemäß)

74 g (0,5 Mol) 4-Methyl-γ-butyrolacton werden in eine Suspension von 27 g (0,50 Mol) Natriummethylat in 400 g Methyl-tert.-butylether gegeben und innerhalb von 1 h mit 68 g (0,5 Mol) Anisaldehyd unter Rühren bei Raumtemperatur versetzt. Man hält den Ansatz noch 1 h unter Rückfluß, kühlt dann ab, versetzt den Ansatz mit 200 g Eiswasser und stellt mit 10 %iger Schwefelsäure auf pH 5 ein. Nach dem Einstellen der Phasentrennung wird das Produkt destilliert. Auf diese Weise entstehen 65 g der gewünschten Verbindung mit einer Ausbeute von 48 % der Theorie. Die spezifische Extinktion E¹/₁ der erfindungsgemäß neuen Verbindung beträgt 1.250, gemessen bei einer Wellenlänge von λₘₐₓ 310 nm.

### Beispiel 2: p-Methoxybenzyliden-4,4-dimethyl-γ-butyrolacton (nicht erfindungsgemäß)

Analoges Verfahren mit 4,4-Dimethyl-y-butyrolacton. E¹/₁ 1.200 (λₘₐₓ 310 nm).

### Beispiel 3: p-Methoxybenzyliden-3,4-dimethyl-γ-butyrolacton (nicht erfindungsgemäß)

Analoges Verfahren mit 3,4-Dimethyl-γ-butyrolacton. E¹/₁ 1.050 (λₘₐₓ 314 nm).

### Beispiel 4: p-Methoxybenzyliden-3,4,4-trimethyl-γ-butyrolacton

Analoges Verfahren mit 3,4,4-Trimethyl-γ-butyrolacton. E¹/₁ 1.060 (λₘₐₓ 312 nm).

### Beispiel 5: p-Methoxybenzyliden-3-ethyl-4,4-dimethyl-γ-butyrolacton

Analoges Verfahren mit 3-Ethyl-4,4-dimethyl-γ-butyrolacton. E¹/₁ 940 (λₘₐₓ 312 nm).

### Beispiel 6: p-Methoxybenzyliden-3-methyl-4-pentyl-γ-butyrolacton (nicht erfindungsgemäß)

Analoges Verfahren mit 3-Methyl-4-pentyl-y-butyrolacton. E¹/₁ 830 (λₘₐₓ 313 nm).

### Beispiel 7: p-Methoxybenzyliden-3,4-dimethyl-4-butyl-γ-butyrolacton

Analoges Verfahren mit 3,4-Dimethyl-4-butyl-γ-butyrolacton. E¹/₁ 810 (λₘₐₓ 312 nm).

### Beispiel 8: p-Methoxybenzyliden-3,4-dimethyl-4-ethyl-y-butyrolacton

Analoges Verfahren mit 3,4-Dimethyl-4-ethyl-y-butyrolacton. E¹/₁ 960 (λₘₐₓ 311 nm).

### 2. Erfindungsgemäße Mittel, deren Herstellung und die Bestimmung des Lichtschutzfaktors:

Die Zusammensetzung sowie die Herstellungsvorschrift verschiedener erfindungsgemäßer Sonnenschutzmittel sind in den Beispielen 9 bis 14 aufgelistet. Dabei bedeutet (O/W), daß es sich zum eine Öl-in-Wasser-Emulsion handelt. Die Abkürzung (W/O) kennzeichnet eine Wasser-in-Öl-Emulsion. Beispiel 15 beschreibt die Zusammensetzung und Herstellung eines Sonnenschutzöls, Beispiel 16 einer Sonnenschutzcreme bzw. eines -Gels und Beispiel 17 offenbart die Bestandteile und Herstellung eines erfindungsgemäßen Haarschampoos.

### Beispiel 9

### Sonnenschutzlotion (O/W)

| | **BESTANDTEILE** | **%** |
|---|---|---|
| A) | Arlatone 983 S | 1,75 |
| | Brij 76 | 1,25 |
| | Lanette O | 1,15 |
| | Myritol 318 | 15,00 |
| | Cetiol SN | 15,00 |
| | Phenonip | 0,20 |
| | UV-Absorber n-Methoxybenzyliden-3,4-dimethyl-4-ethyl-γ-butyrolacton | 5,00 |
| B) | Wasser, dest. | 31,65 |
| | 1,2-Propylenglykol | 2,00 |
| | Phenonip | 0,30 |
| C) | Wasser, dest. | 25,00 |
| | Carbopol 2984 | 0,30 |
| | Natriumhydroxid, 10%ig in Wasser | 1,00 |
| D) | Parfumöl | 0,40 |
| HERSTELLVORSCHRIFT: | | |
| Teil A: Bei ca. 80°C aufschmelzen Teil B: Auf ca. 90°C erhitzen, Teil B unter Rühren zu Teil A geben. Teil C: Carbopol klumpenfrei in Wasser dispergieren, mit Natrium-hydroxid-Lösung zu einem Gel neutralisieren, bei ca. 60°C zu Teil A/B geben. Auf Raumtemperatur abrühren. Teil D: Bei ca. 30°C die Emulsion parfümieren, pH-Wert kontrollieren (6,5 bis 7,0). | | |

### Beispiel 10

### Sonnenschutzmilch (W/O)

| | **BESTANDTEILE** | **%** |
|---|---|---|
| A) | Dehymuls PG PH | 5,00 |
| | Permulgin 3220 | 0,50 |
| | Zinkstearat | 0,50 |
| | Myritol 318 | 15,00 |
| | Cetiol SN | 15,00 |
| | UV-Absorber n-Methoxybenzyliden-3,4-dimethyl-4-ethyl-γ-butyrolacton | 5,00 |
| B) | Wasser, dest. | 52,50 |
| | Glycerin 86 % | 5,00 |
| | Magnesiumsulfat 7 H₂O | 0,50 |
| | Phenonip | 0,50 |
| C) | Parfumöl | 0,50 |
| **HERSTELLVORSCHRIFT:** | | |
| Teil A: Bei ca. 90°C sorgfältig aufschmelzen. Teil B: Auf ca. 95°C erhitzen, dann Teil B unter Rühren zu Teil A geben. Auf Raumtemperatur abrühren. Teil C: Bei 30°C Teil C zugeben und anschließend homogenisieren. | | |

### Beispiel 11

### Sonnenschutzlotion (O/W)

| | **BESTANDTEILE** | **%** |
|---|---|---|
| A) | Arlatone 983 S | 1,75 |
| | Brij 76 | 1,25 |
| | Lanette O | 1,15 |
| | Myritol 318 | 15,00 |
| | Cetiol SN | 15,00 |
| | Finsolv TN | 5,00 |
| | Phenonip | 0,20 |
| | UV-Absorber n-Methoxybenzyliden-3,4-dimethyl-4-ethyl-γ-butyrolacton | 4,00 |
| | Parsol 1789 | 1,50 |
| B) | Wasser, dest. | 26,15 |
| | 1,2-Propylenglykol | 2,00 |
| | Phenonip | 0,30 |
| C) | Wasser, dest. | 25,00 |
| | Carbopol 2984 | 0,30 |
| | Natriumhydroxid, 10%ig in Wasser | 1,00 |
| D) | Parfumöl | 0,40 |
| **HERSTELLVORSCHRIFT:** | | |
| Teil A: Bei ca. 80°C aufschmelzen Teil B: Auf ca. 90°C erhitzen. Teil B unter Rühren zu Teil A geben. Teil C: Carbopol klumpenfrei in Wasser dispergieren, mit Natriumhyroxid-Lösung zu einem Gel neutralisieren, bei ca. 60°C zu Teil A/B geben. Auf Raumtemperatur abrühren. Teil D: Bei ca. 30°C die Emulsion parfümieren, pH-Wert kontrollieren (6,5 bis 7,0). | | |

### Beispiel 12

### Sonnenschutzlotion (O/W)

| | **BESTANDTEILE** | **%** |
|---|---|---|
| A) | Arlatone 983 S | 1,75 |
| | Brij 76 | 1,25 |
| | Lanette O | 1,15 |
| | Myritol 318 | 12,00 |
| | Cetiol SN | 12,00 |
| | UV-Absorber n-Methoxybenzyliden-3,4-dimethyl-4-ethyl-γ-butyrolacton | 7,00 |
| | NEO HELIOPAN® E 1000 | 7,00 |
| | Phenonip | 0,20 |
| B) | Wasser, dest. | 28,65 |
| | 1,2-Propylenglykol | 2,00 |
| | Phenonip | 0,30 |
| C) | Wasser, dest. | 25,00 |
| | Carbopol 2984 | 0,30 |
| | Natriumhydroxid. 10%ig in Wasser | 1,00 |
| D) | Parfumöl | 0,40 |
| **HERSTELLVORSCHRIFT:** | | |
| Teil A: Bei ca. 80°C aufschmelzen Teil B: Auf ca. 90°C erhitzen. Teil B unter Rühren zu Teil A geben. Teil C: Carbopol klumpenfrei in Wasser dispergieren, mit Natriumhyroxid-Lösung zu einem Gel neutralisieren, bei ca. 60°C zu Teil A/B geben. Auf Raumtemperatur abrühren. Teil D: Bei ca. 30°C die Emulsion parftimieren, pH-Wert kontrollieren (6,5 bis 7,0). | | |

### Beispiel 13

### Sonnenschutzlotion (O/W)

| | **BESTANDTEILE** | **%** |
|---|---|---|
| A) | Arlacel 165 | 3,00 |
| | Eumulgin B 2 | 1,00 |
| | Lanette | 1,00 |
| | Myritol 318 | 4,00 |
| | Cetiol OE | 2,00 |
| | Abil 100 | 1,00 |
| | Bentone Gel MIO | 3,00 |
| | Cutina CBS | 1,00 |
| | Phenonip | 0,20 |
| | NEO HELIOPAN® OS (Octyl Salicylate) | 3,00 |
| | NEO HELIOPAN® AV (Octyl Methoxycinnamate) | 5,00 |
| | NEO HELIOPAN® E 1000 (Isoamyl p-Methoxycinnamate) | 5,00 |
| | NEOHELIOPAN® MBC (4-Methylbenzylidene Camphor) | 1,00 |
| | Eusolex TA | 3,00 |
| | UV-Absorber n-Methoxybenzyliden-3,4-dimethyl-4-ethyl-γ-butyrolacton | 3,00 |
| B) | Wasser, dest. | 45,60 |
| | Glycerin, 86%ig | 3,00 |
| | Phenonip | 0,30 |
| | Veegum Ultra | 1,00 |
| | Natrosol 250 HHR | 0,30 |
| | NEO HELIOPAN ® HYDRO, eingesetzt als 15%ige Lösung nach Neutralisation mit Natriumhydroxid (Phenyl benzimidazole Sulfonic Acid) entspricht Aktivsubstanz: 2,0% | 13,30 |
| C) | Pafümöl | 0,30 |
| **HERSTELLVORSCHRIFT:** | | |
| Teil A: Bei ca. 80°C aufschmelzen, dann Eusolex TA sorgfältig dispergieren. Teil B: Ohne Veegum und Natrosol auf ca. 90°C erhitzen, dann Veegum und Natrosol dispergieren, Teil B unter Rühren zu Teil A geben. Auf Raum-temperatur abrühren. Teil C: Bei 30°C Teil C zugeben und anschließend homogenisieren. pH-Wert überprüfen (7,0-7,5). | | |

### Beispiel 14

### Sonnenschutzlotion (W/O)

| | **BESTANDTEILE** | **%** |
|---|---|---|
| A) | Arlacel 1689 | 3,50 |
| | Finsolv TN | 6,00 |
| | NEO HELIOPAN® E 1000 (Isoamyl p-Methoxycinnamate) | 7,00 |
| | Uvinul T 150 (Octyl Triazone) | 1,00 |
| | UV-Absorber n-Methoxybenzyliden-3,4-dimethyl-4-ethyl-γ-butyrolacton | 3,00 |
| | Copherol F 1250 | 2,00 |
| | Permulgin 2550 | 1,00 |
| | Myritol 318 | 6,00 |
| | Cetiol SN | 6,00 |
| | ZINKOXID NEUTRAL H&R (Zinc Oxide) | 7,00 |
| B) | Wasser, dest. | 51,70 |
| | Glycerin 86%ig | 5,00 |
| | Phenonip | 0,50 |
| C) | Parfumöl | 0,30 |
| **HERSTELLVORSCHRIFT:** | | |
| Teil A: Bei ca. 90°C sorgfältig aufschmelzen (ohne ZINKOXID NEUTRAL H&R). Anschließend ZINKOXID NEUTRAL H&R sorfältig dispergie-ren. Teil B: Auf ca. 95°C erhitzen, darm Teil B unter Rühren zu Teil A geben. Auf Raumtemperatur abrühren. Teil C: Bei 30°C Teil C zugeben und anschließend homogenisieren. | | |

### Beispiel 15

### Sonnenschutzöl

| | **BESTANDTEILE** | **%** |
|---|---|---|
| A) | NEO HELIOPAN® E 1000 (Isoamyl p-Methoxycinnamate) | 7,50 |
| | NEO HELIOPAN® OS (Octyl Salicylate) | 5,00 |
| | UV-Absorber n-Methoxybenzyliden-3,4-dimethyl-4-ethyl-γ-butyrolacton | 3,00 |
| | Myritol 318 | 34,70 |
| | Diisopropyladipat | 5,00 |
| | Olivenöl | 1,00 |
| | Jojobaöl | 1,00 |
| | Macadamia Nußöl | 1,00 |
| | Tocopherolöl | 1,00 |
| | Isopropylmyristat | 35,00 |
| | Antaron V-216 | 5,00 |
| | Phenonip | 0,50 |
| | Parfumöl | 0,30 |
| **HERSTELLVORSCHRIFT:** | | |
| Alle Bestandteile sorgfältig vermischen. | | |

### Beispiel 16

### Sonnenschutz Creme Gel

| | **BESTANDTEILE** | **%** |
|---|---|---|
| A) | Wasser, dest. | 75,35 |
| | Phenonip | 0,50 |
| | EDTA B liquid | 0,10 |
| B) | NEO HELIOPAN® AV (Octyl Methoxycinnamate) | 7,00 |
| | NEO HELIOPAN® 303 (Octocrylene) | 3,00 |
| | NEO HELIOPAN® MBC (4-Methylbenzylidene Camphor) | 1,00 |
| | UV-Absorber n-Methoxybenzyliden-3,4-dimethyl-4-ethyl-γ-butyrolacton | 3,00 |
| | Cetiol SN | 5,00 |
| | Eutanol G | 3,00 |
| | Lameform TG I | 1,00 |
| | Parfumöl | 0,30 |
| | Permulen TR-1 | 0,25 |
| | Carbopol 954 | 0,05 |
| C) | Triethanolamin | 0,45 |
| **HERSTELLVORSCHRIFT:** | | |
| Teil A: Inhaltstoffe im Wasser lösen. Teil B: Alle Bestandteile (ohne Permulen und Carbopol) vermischen. NEO HELIOPAN® MBC und UV-Absorber gemäß Formel (I) unter leichtem Erwärmen lösen. Carbopol und Permulen dispergieren. An-schließend Teil B zu Teil A geben und 45 Minuten intensiv verrühren. Teil C: Triethanolamin unter Rühren zu Teil A/B geben. Weiterrühren bis das Produkt homogen ist. pH Wert kontrollieren (ca. 7,0). | | |

### Beispiel 17

### Haarschampoo

| | BESTANDTEILE | % |
|---|---|---|
| A) | Genapol LRO flüssig | 18,00 |
| | Texapon MG3 | 36,00 |
| | Lamepon S | 6,00 |
| | Parfumöl | 0,60 |
| | Phenonip | 0,50 |
| | Arlatone G | 2,00 |
| | UV-Absorber n-Methoxybenzyliden-3,4-dimethyl-4-ethyl-γ-butyrolacton | 0,50 |
| B) | Wasser, dest. | 34,00 |
| | Polymer JR 400 | 0,20 |
| | D-Panthenol | 1,00 |
| | Natriumchlorid | 1,00 |
| | Natriumhydroxid, 10%ig in Wasser | 0,20 |
| **HERSTELLVORSCHRIFT:** | | |
| Teil A: UV-Absorber in NEO HELIOPAN® E 1000 und Phenonip unter schwachem Erwärmen lösen, dann Arlatone G und Parfumöl zugeben und gut vermischen. Restliche Bestandteile einwiegen. Teil B: Polymer unter Rühren im Wasser lösen, restliche Bestandteile zugeben und lösen. Teil B zu Teil A geben und verrühren (pH-Wert kontrollieren, ca. 5,5). | | |

### 3. Handelsübliche zur Herstellung der erfindungsgemäßen Mittel verwendeten Stoffe:

Die unter Punkt 2 zur Herstellung der erfindungsgemäßen Mittel verwendeten, handelsüblichen Stoffe sowie deren Lieferanten sind in den nachfolgenden Übersichten zusammengefaßt.

| **Handelsname** | **Chemische Bezeichnung** | **Lieferant** |
|---|---|---|
| Abil 100 | Polydimethylsiloxan | 7 |
| Antaron V-216 | Vinylpyrrolidon/Hexadecen Copolymer | 18 |
| Arlacel 1689 | Sorbitan Monooleat/Propylglyceryl-3-ricinoleat | 4 |
| Arlacel 165 | Glycerinstearat/Polyethylenglykol-(MG 100)-Stearat-Mischung | 4 |
| Arlatone G | mit 25 Mol Ethylenoxid gehärtetes | 4 |
| Arlatone 983 S | Polyethylenglykol-(MG 5)-Glycerylstearat | 4 |
| Baysilone Fluid PK 20 | Siliconöl | 5 |
| Betone Gel MIO | Mineralöl, Quaternium-18 Hectorit, Propylencarbonat | 17 |
| Brij 76 | Polyethylenglycol (MG 10) Stearyl-Ether | 4 |
| Carbopol 2984 | Polyacrylsäure | 2 |
| Carbopol 954 | Polyacrylsäure | 2 |
| Cetiol HE | Polyol-Fettsäure-Ester | 3 |
| Cetiol OE | Dicaprylylether | 3 |
| Cetiol SN | Cetyl-/Stearyl-isononanoat | 3 |
| Copherol F 1250 | D-α-Tocopherylacetat | 3 |
| Cutina CBS | Glycerinstearat, Cetyl-/Stearylalkohol, Cetylpalmitat, Kokosnußglyceride | 3 |
| | | |
| Dehymuls PG PH | Polyglycerinpoly-12-hydroxystearat | 3 |
| Diisopropyladipat | Adipinsäurediisopropylester | 3 |
| D-Panthenol | Panthothenyl-Alkohol | 15 |
| EDTA B fl. | Tetranatrium-ethylendiamintetraessigsäure | 6 |
| Eusolex TA | Titandioxid | 13 |
| Eutanol G | 2-Octyldodecanol | 3 |
| Eumulgin B2 | Cetyl-/Stearylalkohol, verethert mit 20 Mol Ethylenoxid | 3 |
| Finsolv TN | Alkylbenzoat | 23 |
| Genapol LRO fl. | Natriumlaurylsulfat | 9 |
| Glycerin | 1,2,3-Propantriol | 3 |
| Isopropylmyristat | Myristinsäureisopropylester | 3 |
| Jojobaöl | Jojobaöl | 19 |
| Lameform TGI | Triglycerindiisostearat | 3 |
| Lamepon S | Eiweiß/Kokosfettsäure-Kondensat, Kaliumsalz | 3 |
| Lanette O | Cetyl-/Stearylalkohol-Mischung | 3 |
| Macadamia-Nußöl | Macadamia-Nußöl | 20 |
| Myritol 318 | Capryl-/Caprin-triglycerid | 3 |
| Natrosol 250 HHR | Hydroxyethylcellulose | 11 |
| NEO HELIOPAN®AV | p-Methoxyzimtsäureisooctylester | 1 |
| NEO HELIOPAN®BB | 2-Hydroxy-4-methoxybenzophenon | 1 |
| NEO HELIOPAN®E 1000 | p-Methoxyzimtsäureisoamylester | 1 |
| NEO HELIOPAN® HYDRO | Phenylbenzimidazolsulfonsäure | 1 |
| NEO HELIOPAN® MBC | 3-(4-Methylbenzylidene)-d, 1-Campher | 1 |
| NEO HELIOPAN®OS | 1-Ethylhexylsalicylat | 1 |
| | | |
| NEO HELIOPAN®303 | α-Phenyl-ß-cyan-zimtsäureisooctylester | 1 |
| Olivenöl | Olivenöl | 21 |
| Parsol 1789 | Butyl Methoxydibenzoylmethan | 12 |
| Permulgin 2550 | Wachs | 14 |
| Permulgin 3220 | Wachs | 14 |
| Permulen TR 1 | Polyacrylat | 2 |
| Phenonip | Gemisch von p-Hydroxybenzoe-säureestern und Phenoxyethanol | 8 |
| Polymer JR 400 | Polyquaternium-10 | 21 |
| 1,2-Propylenglykol | 1,2-Propandiol | 6 |
| Texapon MG 3 | Magnesiumlaurylsulfat/Dinatriumlaurylsulfosuccinat | 3 |
| Tocopherolöl | Sojaöl mit D-α-Tocopherol | 22 |
| Uvinul T 150 | Triazinyl-p-aminobenzoesäureisooctylester | 6 |
| Veegum Ultra | Magnesiumaluminiumsilikat | 10 |
| ZINC OXIDE NEUTRAL H&R | Zinkoxid | 1 |
| Zinkstearat | Zinkstearat | 16 |

### Lieferanten

| | |
|---|---|
| 1. | Haarmann & Reimer GmbH, Holzminden |
| 2. | B.F. Goodrich Company, Neuss |
| 3. | Henkel KGaA, Düsseldorf |
| 4. | ICI Speciality Chemicals, Frankfurt |
| 5. | Bayer AG, Leverkusen |
| 6. | BASF, Ludwigshafen |
| 7. | Goldschmidt AG, Essen |
| 8. | Nipa Lab. Ltd., Pontypridd Mid Glam, Wales / GB |
| 9. | Hoechst AG, Frankfurt |
| 10. | R.T. Vanderbilt Company Inc., Norwalk / USA |
| 11. | Hercules Inc., Wilmington, Delaware/USA |
| 12. | Hoffmann-LaRoche, Basel/CH |
| 13. | E. Merck, Darmstadt |
| 14. | Koster Keunen Holland BV, Bladl /NL |
| 15. | Akzo Chemie GmbH, Düren |
| 16 | Chemische Werke Bärlocher, München |
| 17. | Rheox Inc., Hightstown, New Jersey / USA |
| 18. | ISP Global Technologies Deutschland GmbH, Frechen |
| 19. | Henry Lamotte, Bremen |
| 20 | Erhard Wagner GmbH, Bremen |
| 21. | Nordmann & Rassmann GmbH & Co., Hamburg |
| 22 | Richter GmbH, Berlin |
| 23. | Witco Surfactants GmbH, Steinau a.d. Straße |

## Patentansprüche

1. Benzyliden-γ-butyrolacton der allgemeinen Formel 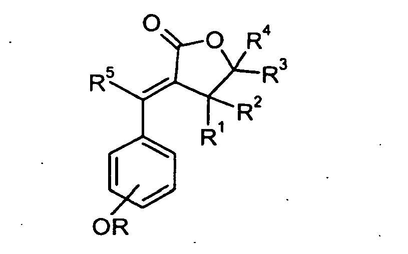 wobei
R Wasserstoff oder C₁-C₆-Alkyl oder Cycloalkyl ist und
R² und R⁵ unabhängig voneinander Wasserstoff oder C₁-C₈-Alkyl oder Cycloalkyl sind
und
der
Lactonring trisubstituiert ist (R¹, R³ und R⁴ = C₁-C₈-Alkyl), wobei eine Alkylgruppe mindestens eine C₂-Kette beinhaltet.

2. Benzyliden-γ-butyrolacton nach Anspruch 1, **dadurch gekennzeichnet, daß** der Benzylidenring in para-Stellung mit einem Methoxy-Rest substituiert ist und R¹ Ethyl, R² Wasserstoff und R³/R⁴ Methyl ist.

3. Mittel enthaltend Benzyliden-γ-butyrolacton nach einem der Ansprüche 1 bis 2, **dadurch gekennzeichnet, daß** es ein oder mehrere Benzyliden-γ-butyrolactone enthält.

4. Mittel nach Anspruch 3, **dadurch gekennzeichnet, daß** es 0,1 bis 15 Gew.-% an Benzyliden-γ-butyrolacton, bezogen auf das Gesamtgewicht der Zubereitung, enthält.

5. Mittel nach einem der Ansprüche 3 oder 4, **dadurch gekennzeichnet, daß** es 1 bis 10 Gew.-% an Benzyliden-γ-butyrolacton, bezogen auf das Gesamtgewicht der Zubereitung, enthält.

6. Mittel nach einem der Ansprüche 3 bis 5, **dadurch gekennzeichnet, daß** es 2 bis 7 Gew.-% an Benzyliden-γ-butyrolacton, bezogen auf das Gesamtgewicht der Zubereitung, enthält.

7. Mittel nach einem der Ansprüche 3 bis 6, **dadurch gekennzeichnet, daß** es ein UV-Absorber und gleichzeitig ein Lösungsmittel ist.

8. Mittel nach einem der Ansprüche 3 bis 7, **dadurch gekennzeichnet, daß** es weitere an sich bekannte Stoffe und/oder UV-Absorber und/oder Pigmente enthält.

9. Mittel nach einem der Ansprüche 3 bis 8, **dadurch gekennzeichnet, daß** es als Emulsion, Milch, Lotion, Creme, Gel, Aerosol, Shampoo, Spülung, Kur, Spray oder jegliche andere übliche kosmetische oder pharmazeutische Zubereitung vorliegt.

10. Mittel nach einem der Ansprüche 3 bis 9, **dadurch gekennzeichnet, daß** es farblos, geruchsneutral und wasserfest ist.

11. Verfahren zur Herstellung von Mitteln nach einem der Ansprüche 3 bis 10, **dadurch gekennzeichnet, daß** Benzyliden-γ-butyrolactone mit weiteren an sich bekannten Stoffen und/oder UV-Absorbern und/oder Pigmenten vermischt werden.

12. Verwendung der Mittel nach einem der Ansprüche 3 bis 10 als Sonnenschutz- oder Tagespflegemittel zum Schutz von menschlicher Haut und Haar vor schädigender UV-Strahlung.

13. Verwendung der Mittel nach einem der Ansprüche 3 bis 10 zum Schutz technischer Produkte, wie Anstriche, Lacke, Kunststoffe, Textilien, Verpackungsmaterialien oder Kautschuk, vor schädigender UV-Strahlung.

14. Verwendung der Mittel nach einem der Ansprüche 3 bis 10 als Lösungsmittel für schlechter lösliche feste UV-Absorber innerhalb einer kosmetischen Zubereitung.

## Claims

1. Benzylidene-γ-butyrolactone of the general formula 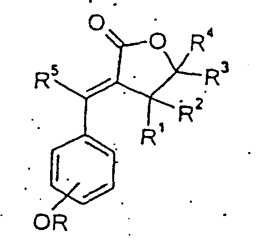 wherein
R is hydrogen or C₁-C₆ alkyl or cycloalkyl, and
R² and R⁵ are mutually independently hydrogen or C₁-C₈ alkyl or cycloalkyl
and the lactone ring is trisubstituted (R¹, R³ and R⁴ = C₁-C₈ alkyl), wherein an alkyl group includes at least one C₂ chain.

2. Benzylidene-γ-butyrolactone according to claim 1, **characterised in that** the benzylidene ring is substituted in the para-position by a methoxy radical and R¹ is ethyl, R² is hydrogen and R³/R⁴ is methyl.

3. Agent containing benzylidene-γ-butyrolactone according to one of claims 1 to 2, **characterised in that** it contains one or more benzylidene-γ-butyrolactones.

4. Agent according to claim 3, **characterised in that** it contains 0.1 to 15 wt.% of benzylidene-γ-butyrolactone, based on the total weight of the preparation.

5. Agent according to one of claims 3 or 4, **characterised in that** it contains 1 to 10 wt.% of benzylidene-γ-butyrolactone, based on the total weight of the preparation.

6. Agent according to one of claims 3 to 5, **characterised in that** it contains 2 to 7 wt.% of benzylidene-γ-butyrolactone, based on the total weight of the preparation.

7. Agent according to one of claims 3 to 6, **characterised in that** it is a UV absorber and also a solvent.

8. Agent according to one of claims 3 to 7, **characterised in that** it contains further substances known per se and/or UV absorbers and/or pigments.

9. Agent according to one of claims 3 to 8, **characterised in that** it is in the form of an emulsion, milk, lotion, cream, gel, aerosol, shampoo, conditioner, intensive conditioner, spray or any other conventional cosmetic or pharmaceutical preparation.

10. Agent according to one of claims 3 to 9, **characterised in that** it is colourless, neutral in odour and water-resistant.

11. Process for the preparation of agents according to one of claims 3 to 10, **characterised in that** benzylidene-γ-butyrolactones are mixed with further substances known per se and/or UV absorbers and/or pigments.

12. Use of the agents according to one of claims 3 to 10 as a sunscreen or daycare product for protecting human skin and hair against harmful UV radiation.

13. Use of the agents according to one of claims 3 to 10 for protecting industrial products, such as paints, surface coatings, plastics, textiles, packaging materials or rubber, against harmful UV radiation.

14. Use of the agents according to one of claims 3 to 10 as a solvent for solid UV absorbers which have poor solubility, within a cosmetic preparation.

## Revendications

1. Benzylidène-γ-butyrolactone de formule générale 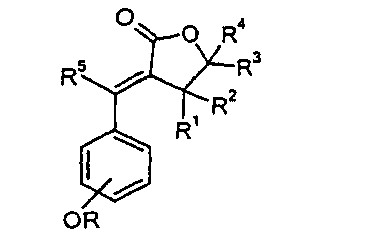 dans laquelle
R est un atome d'hydrogène ou un groupe alkyle en C₁ à C₆ ou cycloalkyle, et
R² et R⁵ sont, indépendamment l'un de l'autre, un atome d'hydrogène ou un groupe alkyle en C₁ à C₈ ou cycloalkyle, et
le cycle lactone est trisubstitué (R¹, R³ et R⁴ = un groupe alkyle en C₁ à C₈), où un groupe alkyle comprend au moins une chaîne en C₂.

2. Benzylidène-γ-butyrolactone selon la revendication 1, **caractérisée en ce que** le cycle benzylidène est substitué par un radical méthoxy en position para et que R¹ est un groupe éthyle, R² est un groupe hydrogène et R³/R⁴ est un groupe méthyle.

3. Agent contenant de la benzylidène-γ-butyrolactone selon l'une des revendications 1 à 2, **caractérisée en ce qu'**il comprend une ou plusieurs benzylidène-γ-butyrolactones.

4. Agent selon la revendication 3, **caractérisé en ce qu'**il comprend 0,1 à 15 % en poids de benzylidène-γ-butyrolactone, par rapport au poids total de la composition.

5. Agent selon l'une des revendications 3 ou 4, **caractérisé en ce qu'**il comprend 1 à 10 % en poids de benzylidène-γ-butyrolactone, par rapport au poids total de la composition.

6. Agent selon l'une des revendications 3 à 5, **caractérisé en ce qu'**il comprend 2 à 7 % en poids de benzylidène-γ-butyrolactone, par rapport au poids total de la composition.

7. Agent selon l'une des revendications 3 à 6, **caractérisé en ce qu'**il est un absorbeur d'UV et en même temps un solvant.

8. Agent selon l'une des revendications 3 à 7, **caractérisé en ce qu'**il comprend d'autres substances connues en soi et/ou absorbeurs d'UV et/ou pigments.

9. Agent selon l'une des revendications 3 à 8, **caractérisé en ce qu'**il se présente sous forme d'émulsion, de lait, de lotion, de crème, de gel, d'aérosol, de shampooing, d'après-shampooing, de cure, de spray ou de toute autre composition cosmétique ou pharmaceutique usuelle.

10. Agent selon l'une des revendications 3 à 9, **caractérisé en ce qu'**il est incolore, sans odeur et résiste à l'eau.

11. Procédé de préparation d'agents selon l'une des revendications 3 à 10, **caractérisé en ce que** les benzylidène-γ-butyrolactones sont mélangées avec d'autres substances connues en soi et/ou absorbeurs d'UV et/ou pigments.

12. Utilisation des agents selon l'une des revendications 3 à 10 en tant qu'agents photoprotecteurs ou agents de soins quotidiens pour protéger la peau humaine et les cheveux des rayons ultraviolets nocifs.

13. Utilisation des agents selon l'une des revendications 3 à 10 pour protéger des rayons ultraviolets nocifs les produits techniques tels que les peintures et vernis, les laques, les matières plastiques, les textiles, les matériaux d'emballage ou le caoutchouc.

14. Utilisation des agents selon l'une des revendications 3 à 10 en tant que solvant pour des absorbeurs d'UV solides difficilement solubles dans une composition cosmétique.
